# EUROPEAN PATENT SPECIFICATION

(11) **EP 4 125 858 B1**
(45) Date of publication and mention of the grant of the patent: **06.05.2026**
(21) Application number: 21705032.7
(22) Date of filing: 19.01.2021
(51) Int. Cl.: A61K 31/205, A23L 33/175, A61P 31/14, A23L 33/10

(54) **NUTRITIONAL COMPOSITION COMPRISING L-CARNITINE TARTRATE FOR USE IN TREATING OR PREVENTING A CORONAVIRUS INFECTION IN A MAMMAL**
NÄHRSTOFFZUSAMMENSETZUNG ENTHALTEND L-CARNITINTARTRAT ZUR VERWENDUNG BEI DER BEHANDLUNG ODER PRÄVENTION EINER CORONAVIRUSINFEKTION BEI EINEM SÄUGER
COMPOSITION NUTRITIONNELLE COMPRENANT DU TARTRATE DE L-CARNITINE POUR UNE UTILISATION DANS LE TRAITEMENT OU LA PRÉVENTION D'UNE INFECTION PAR CORONAVIRUS CHEZ UN MAMMIFÈRE

(30) Priority: 28.04.2020 US 202063016579 P; 22.05.2020 US 202063028934 P; 12.11.2020 US 202063112942 P
(43) Date of publication of application: 08.02.2023
(73) Proprietor: Lonza Greenwood LLC, Greenwood, SC 29646 (US)
(72) Inventor: BELLAMINE, Aoutef, Morristown, New Jersey 07960 (US); DURKEE, Shane, Morristown, New Jersey 07960 (US)
(74) Representative: Greiner, Elisabeth
(86) International application number: PCT/US2021/013921
(87) International publication number: WO 2021/221741

(56) References cited:
- WO-A1-02/40013
- WO-A1-2005/110112
- WO-A1-2007/003481
- WO-A1-2018/227228
- WO-A2-97/05862
- WO-A2-97/05864
- CN-A- 1 879 788
- KR-B1- 101 355 018
- US-A1- 2006 135 422
- YESUDHAS DHANUSHA ET AL: "COVID-19 outbreak: history, mechanism, transmission, structural studies and therapeutics", INFECTION, vol. 49, no. 2, September 2020 (2020-09-01), pages 199 - 213, XP037392916, ISSN: 0300-8126, DOI: 10.1007/S15010-020-01516-2
- FAKHROLMOBASHERI MOHAMMAD ET AL: "L-Carnitine can extinguish the COVID19 fire: A review on molecular aspects", 4 April 2020 (2020-04-04), pages 1 - 22, XP055795861, Retrieved from the Internet <URL:https://zenodo.org/record/3740145/files/Manuscript.pdf?download=1> [retrieved on 20210415], DOI: 10.5281/zenodo.3740145

## Description

The present application claims priority to U.S. Provisional Application Serial No. 63/016,579, filed on April 28, 2020, U.S. Provisional Application Serial No. 63/028,934, filed on May 22, 2020, and U.S. Provisional Application Serial No. 63/112,942 filed on November 12, 2020.

### BACKGROUND

L-carnitine was first discovered in 1905. Since then, extensive research has demonstrated the important role it plays in helping bodies utilize dietary fat for energy. Without sufficient L-carnitine in the body, humans and animals would not be able to utilize fat effectively and long-term health problems may occur.

In the body, L-carnitine is known to shuttle long-chain fatty acids across the inner-mitochondrial membrane so that the fatty acids can be metabolized and converted into energy. Without L-carnitine, these fatty acids would not be transported and properly utilized.

Mammals can obtain L-carnitine naturally from two sources. First, L-carnitine can be biosynthesized within the body. The body, however, can only produce small amounts of L-carnitine. L-carnitine can also be obtained from foods, such as red meat.

The assignee of the present disclosure markets a L-carnitine and salts thereof as nutritional supplements for mammal application. In the past, L-carnitine and derivatives and salts thereof have been administered to mammals. For instance, L-carnitine and derivatives and/or salts thereof can be administered to a mammal when the diet of the mammal is low in L-carnitine. L-carnitine and derivatives and salts thereof have also been administered to mammals in order to prevent obesity, for heart health and other various health reasons. L-carnitine and derivatives and salts thereof have also been marketed to older or senior pets. As mammals increase in age, for instance, the availability of L-carnitine in the body decreases because food intake normally decreases and the body's ability to produce L-carnitine begins to drop. Supplementation with L-carnitine and derivatives and/or salts thereof has shown a positive influence on the aging process.

Severe acute respiratory syndrome coronavirus (SARS-CoV-1 and SARS-CoV-2) has been responsible for the SARS epidemic in 2002 to 2004 and more recently for the coronavirus disease 2019 (Covid-19) pandemic outbreak initially detected in December 2019. The disease is caused by the SARS-CoV-2, an airborne virus that affects mainly the lungs and the upper respiratory system, leading ultimately to lung injury, respiratory distress and death in severe cases.

Coronaviruses (CoVs) are enveloped positive-sense RNA viruses, and are characterized by club-like spikes that project from their surface, an unusually large RNA genome, and a unique replication strategy. These spike projection contains protein domains responsible for its interaction with host cells and its entry in these cells. Coronaviruses cause a variety of diseases in mammals and birds ranging from enteritis in cows and pigs and upper respiratory disease chickens to potentially lethal human respiratory infections.

The presence of a novel coronavirus pandemic in 2019 (COVID-19) has posed threat to the world population of humans. A recent study has confirmed that severe acute respiratory syndrome coronavirus 2 (SARS-CoV-2) uses a severe acute respiratory syndrome coronavirus (SARS-CoV) receptor, the angiotensin-converting enzyme 2 (ACE-2) of the host cell, to attach to the host mammal. See Hoffmann M, Kleine-Weber H, Schroeder S, Kruger N, Herrler T, Erichsen S, Schiergens TS, Herrler G, Wu NH, Nitsche A, et al. SARS-CoV-2 Cell Entry Depends On ACE-2 And TMPRSS2 And Is Blocked By A Clinically Proven Protease Inhibitor. Cell. 2020; 181:1-10. ACE-2 expression was previously found to correlate with increase susceptibility to SARS-CoV infection in vitro. Hofmann H, Geier M, Marzi A, Krumbiegel M, Peipp M, Fey GH, Gramberg T, Pohlmann S., Susceptibility To SARS Coronavirus S Protein-Driven Infection Correlates With Expression Of Angiotensin Converting Enzyme 2 And Infection Can Be Blocked By Soluble Receptor. Biochem Biophys Res Commun. 2004; 319:1216-1221. As with SARS-CoV-2, higher ACE-2 expression might also lead to higher risk of COVID-19 infection.

Following its binding to the ACE-2 receptor, the SARS-CoV-2 spike protein is further cleaved to facilitate its entry to the host cell. Cleavage is believed to be mediated by TMPRSS2 (transmembrane protease serine 2) at the position S1/S2 and Furin at the S' position.

Further, a Furin-like cleavage site has been discovered in the spike protein of the SARS-CoV-2, that is not present in other SARS-like CoVs. It is believed that Furin also plays a part in the a Coronavirus 2019 infection. B. Coutarda, et al., The Spike Glycoprotein of the New Coronavirus 2019-nCoV Contains a Furin-like Cleavage Site Absent in CoV of the Same Clade, Antiviral Research 176 (2020) 104742.

Other coronaviruses, such as the Middle East Respiratory Syndrome-CoV (MERS-CoV) utilizes Dipeptidyl peptidase 4 (DPP-4) as its receptor. However, this virus is only able to use the receptor from certain species such as bats, humans, camels, rabbits, and horses to establish infection. As with elevated ACE-2 levels in a mammal, elevated levels of DPP-4 in a mammal may make the mammal more susceptible a MERS-CoV infection.

The physiological role of ACE-2 is to act as an anti-inflammatory mediator as part of the renin-angiotensin system (RAS), and by converting the pro-inflammatory Angiotensin II to the ant-inflammatory Angiontesin-(1-7). Angiotensin II is generated by ACE-1, which is also part of the RAS system. Therefore, keeping the balance between ACE-1 and ACE-2 is essential when considering therapies targeting ACE-2, particularly as the spike in inflammation and subsequent cytokine storm have been reported in advanced diseased COVID-19 patients

There is a need for an effective way to reduce or control ACE-2 expression in mammals infected with, and/or could become infected with an acute respiratory syndrome infection that may propagate in the mammal through the ACE-2 receptor by reducing the presence of the ACE-2 receptor. Since the increase presence of ACE-2 in the mammal may result in an increase of a potential infection or severity of an infection in the mammal from an infectious agent which binds to a receptor of an angiotensin-converting enzyme 2, by reducing or controlling the amount of the ACE-2 present in a mammal, the number of receptors capable of binding to the infectious agent will be reduced, as will the severity of infectious diseases such as viruses and other respiratory infections that use receptors on the ACE-2 to enter the host mammal.

Likewise, there is a need to control the TMPRSS2 (transmembrane protease serine 2) and Furin present in a mammal that may become infected, or is infected with the SARS-CoV-2 virus.

### SUMMARY OF THE DISCLOSURE

In general, the present disclosure provides newly discovered advantages of using carnitine nutritional supplementation in mammals which can reduce the probability the mammal will be rendered ill by an infectious disease that binds to receptors on the angiotensin-converting enzyme 2 (ACE-2).

In one aspect of the present disclosure, provided is L-carnitine tartrate for use in a method for reducing the possibility of infection in a mammal from a coronavirus infectious agent which binds a receptor of an angiotensin-converting enzyme 2 (ACE-2) of the mammal, the method comprising administering L-carnitine tartrate to a mammal in a therapeutically effective amount to reduce the amount of the angiotensin-converting enzyme 2 (ACE-2) in the mammal.

In any further disclosed aspect, any reference to a method is to be read as the ACE-reducing agent for use in said method, wherein the ACE-reducing agent is L-carnitine tartrate and wherein the virus infection is a coronavirus infection.

In a different aspect, disclosed is a method for treating an infection in a mammal caused by an infectious agent which binds a receptor of an angiotensin-converting enzyme 2 (ACE-2) in the mammal. The method has the step of administering an ACE-2 reducing component to the mammal infected with an infectious agent. The ACE-2 reducing component is administered in a therapeutically effective amount to reduce the amount of the ACE-2 present in the mammal.

In another aspect, disclosed is a method for reducing an angiotensin-converting enzyme 2 (ACE-2) level in a mammal having an elevated level of the ACE-2 as result of inflammation caused by an infectious agent or other disease conditions. The method comprises administering an ACE-2 reducing component to the mammal in need of reduction of the ACE-2 levels, the ACE-2 reducing component being administered in a therapeutically effective amount to reduce the amount of the ACE-2 in the mammal to bring the ACE-2 levels closer to the basal level prior to the inflammation.

In further aspect, disclosed is a method for maintaining an angiotensin-converting enzyme 2 (ACE-2) level in a mammal exposed to an infectious agent which binds to the ACE-2 receptor. The method comprises administering ACE-2 reducing component to a mammal in a therapeutically effective amount to maintain the amount of the ACE-2 in the mammal at a level for a heathy mammal to prevent infections agent attachment and propagation of the infection in the mammal.

In yet another aspect of the present disclosure, provided is an angiotensin-converting enzyme 2 (ACE-2) reducing nutritional supplement, containing an ACE-2 reducing component for the use in the treatment, prevention, and/or delay of progression of infection in a mammal caused by an infectious agent which binds to the (ACE-2) in the mammal, wherein the infectious agent comprises a corona virus, wherein the ACE-2 reducing component is L-carnitine tartrate, and wherein said nutritional supplement contains a) L-carnitine tartrate; and b) an auxiliary substance. L-Carnitine tartrate is used in an effective amount to treat, prevent, and/or delay of progression of infection in a mammal caused by an infectious agent, which binds to the (ACE-2).

In a further aspect of the present disclosure, any of the above aspects the nutritional supplement may further comprise acetyl L-carnitine, valeryl L-carnitine, isovaleryl L-carnitine, benzyl L-carnitine, L-leucyl L-carnitine, L-valyl L-carnitine, other L-amino acyl carnitines, salts of L-amino acyl L-carnitine, L-carnitine HCL, L-carnitine fumarate, propionyl L-carnitine, L-carnitine phosphate, acetyl L-carnitine L-aspartate, acetyl L-carnitine citrate, acetyl L-carnitine maleate, acetyl L-carnitine phosphate, acetyl L-carnitine fumarate, propionyl L-carnitine orotate, acetyl L-carnitine orotate, butyryl L-carnitine orotate, propionyl L-carnitine fumarate, L-carnitine oxalate, L-carnitine sulfate, GPLC glycine propionyl L-carnitine or a mixture thereof. In any of the above aspects the ACE-2 reducing component is L-carnitine tartrate.

In a further aspect of the present disclosure, the ACE-2 reducing component may be administered to the mammal orally, nasally, intravenously, intramuscularly or transdermally.

In a further aspect of the present disclosure, the ACE-2 reducing component is administered to a mammal which has been exposed to or is infected with an infection from an infectious agent which binds to a receptor of ACE-2, and the infectious agent is coronavirus and in particular SARS-CoV and the disease COVID -19.

In yet another aspect of the present disclosure, the ACE-2 reducing component is administered to a mammal in combination with at least one additional component which is effective for treating an infectious agent and the underlying disease.

In another aspect, the ACE-2 reducing component is administered at least to the mammal once or twice a day. The ACE-2 reducing component may be administered in an amount between about 5mg and 10,000 mg per day, in particular in an amount between about 250 mg and 5000 mg per day.

It is further contemplated that the present disclosure provides for L-carnitine tartrate for use to treat or prevent an infection disease caused by an infections agent which binds to the ACE-2 receptors, wherein the infectious agent is a coronavirus.

The present disclosure further provides for the control of TMPRSS2 and Furin in addition to ACE-2.

The present invention further avoids or mitigates a cytokine storm caused by an infectious agent, by reducing the ACE-1 or maintaining ACE-1 to maintain or reduced the ACE-1 to ACE-2 ratio to avoid a cytokine storm.

### BRIEF DESCRIPTION OF DRAWINGS

FIGURE 1 shows the effects of L-carnitine supplementation on ACE-2 levels in the liver samples in rodents determined using a western blotting test.
FIGURE 2 shows the effects of L-carnitine supplementation on ACE-2 levels in the muscle samples in rodents determined using a western blotting test.
FIGURE 3 shows the effects of L-carnitine supplementation on ACE-2 fold change in expression levels in the rodent liver samples determined from a mRNA quantification test.
FIGURE 4 shows the effects of L-carnitine supplementation on ACE-2 fold change in expression levels in the rodent muscle samples determined from a mRNA quantification test.
FIGURE 5A shows the effects of L-carnitine supplementation levels of serum ACE-1 as determined by ELISA test.
FIGURE 5B shows the effects of L-carnitine supplementation levels of serum ACE-2 as determined by ELISA test.
FIGURE 5C shows the effects L-carnitine supplementation levels of serum ACE1/ACE-2 ratio as determined by ELISA test.
FIGURE 5D shows the effects L-carnitine supplementation in levels of serum TMPRSS2 as determined by ELISA test.
FIGURE 6A shows L-carnitine effect on ACE-1 in rodent lung tissue.
FIGURE 6B shows L-carnitine effect on ACE-2 in rodent lung tissue.
FIGURE 6C shows L-carnitine effect on TMPRSS2 in rodent lung tissue in terms of percentage of control.
FIGURE 6D shows L-carnitine effect on Furin in rodent lung tissue in terms of percentage of control.
FIGURE 7A shows the fold change of the L-Carnitine effect on ACE-1 in rodent lung tissue.
FIGURE 7B shows the fold change of the L-Carnitine effect on ACE-2 in rodent lung tissue.
FIGURE 7C shows the fold change of the L-Carnitine effect on TMPRSS2 in rodent lung tissue.
FIGURE 7D shows the fold change of the L-Carnitine effect on Furin in rodent lung tissue.
FIGURE 8A shows the L-Carnitine effect on Furin in rodent liver tissue.
FIGURE 8B shows the fold change in Furin based on the L-Carnitine in rodent liver tissue.
FIGURE 9 shows the L-Carnitine effect on ACE-1 in human blood serum from example 4.
FIGURE 10 shows the L-Carnitine effect on ACE-2 in human blood serum from example 4.
FIGURE 11 shows the L-Carnitine effect on TMPRSS2 in human blood serum from example 4.
FIGURE 12 shows the L-Carnitine effect on Furin in human blood serum from example 4.
FIGURE 13 shows the L-Carnitine effect on the ratio ACE-1/ACE-2 in human blood serum from example 4.
FIGURE 14 shows the L-Carnitine effect on Serum CRP in human blood serum from example 4.
FIGURE 15 shows the L-Carnitine effect on TNF-alpha in human blood serum from example 4.
FIGURE 16 shows the cytotocixity Assay in Calu-3 for carnitine.
FIGURE 17A shows the Dose-dependent effect of L-carnitine on ACE2 in Calu-3 cells.
FIGURE 17B shows the dose-dependent effect of L-carnitine on TMPRSS2 in Calu-3 cells.
FIGURES 18A, 18B and 18C show the dose-dependent effect of L-carnitine on ACE-2 surface protein in Calu-3 cells at 100 µM, 500 µM and 1000 µM of the L-Carnitine, respectively.
FIGURES 19A, 19B and 19C show the dose-dependent effect of D-carnitine on ACE-2 surface protein in Calu-3 cells at 100 µM, 500 µM and 1000 µM of the D-Carnitine, respectively.
FIGURE 20 shows the dose-dependent inhibition rate of infection of Calu-3 infection by L-carnitine.
FIGURE 21 shows the 24 hour post infection results of the infection test at 10⁻²
FIGURE 22 shows the 48 hour post infection results of the infection test at 10⁻² dilution.

### DEFINITIONS

As used herein, the terms "about," "approximately," or "generally," when used to modify a value, indicates that the value can be raised or lowered by 10% and remain within the disclosed aspect.

The term "therapeutically effective amount" as used herein, shall mean that dosage, or amount of a composition, that provides the specific pharmacological or nutritional response for which the composition is administered or delivered to mammals in need of such treatment. It is emphasized that "therapeutically effective amount", administered to a particular subject in a particular instance, will not always be effective in treating the ailments or otherwise improve health as described herein, even though such dosage is deemed a "therapeutically effective amount" by those skilled in the art. Specific subjects may, in fact, be "refractory" to a "therapeutically effective amount". For example, a refractory subject may have a low bioavailability or genetic variability in a specific receptor, a metabolic pathway, or a response capacity such that clinical efficacy is not obtainable. It is to be further understood that the composition, or nutritional supplement, in particular instances, can be measured as oral dosages, or with reference to ingredient levels that can be measured in blood. In other embodiments, dosages can be measured in amounts applied to the skin when the composition is contained with a topical formulation.

The term "nutritional supplement" refers to any compound added to a dietary source (e.g., a food, beverage, or a dietary supplement) that provides health or medical benefits in addition to its basic nutritional value as described herein.

The term "administering" as used herein, refers to any route for providing the composition, product, or a nutraceutical, to a subject as accepted as standard by the medical community. For example, the present disclosure contemplates routes of administering that include oral ingestion plus any other suitable route of delivery including transdermal, intravenous, intraperitoneal, intramuscular, topical and subcutaneous.

As used herein, the term "mammal" includes any mammal without limitation human, canine, equine, feline, bovine, ovine, or porcine mammals and other similar species.

As used herein, the term "avian" includes any of warm-blooded vertebrates constituting the class Aves, characterized by feathers, toothless beaked jaws, the laying of hard-shelled eggs, a high metabolic rate, a four-chambered heart, and a strong yet lightweight skeleton. Examples of avian include production birds, such as chickens, turkeys, pheasants, ducks, geese and the like.

As used herein, "healthy" refers to the absence of illness or injury,

Other features and aspects of the present disclosure are discussed in greater detail below.

### DETAILED DESCRIPTION

It is to be understood by one of ordinary skill in the art that the present discussion is a description of exemplary embodiments only, and is not intended as limiting the broader aspects of the present disclosure.

The angiotensin-converting enzyme 2 (ACE-2) has beneficial physiological role in regulating blood pressure in the body of a mammal. ACE-2 levels may be raised as a result of various factors, including, general health of the mammal, including heart disease, high blood pressure and the like, or may be the result of elevating events, for example exercise, inflammation and oxidative stress. A mammal with an elevated levels of ACE-2, is more susceptible to infections from an infectious agent which binds a receptor of an angiotensin-converting enzyme 2, such as the SARS CoV viruses.

It has been discovered that controlling the ACE-2 in a mammal, which has been infected with an infectious agent that binds to or reduce the ACE-2 receptor expression, can be a beneficial treatment for the mammal infected with an infectious agent which can bind to or reduce the ACE-2 receptor. Reducing the ACE-2 in the mammal can lead to can help decrease the number of receptors capable of binding to the infectious agent, which in turn may reduce the severity of infectious diseases caused by viruses and other respiratory infections that use receptors on the ACE-2 to enter the host mammal. In the present disclosure, the reduction and maintaining the ACE-2 level near normal levels when therapeutically effective amounts of carnitine is administered to a mammal under an inflammation state.

Suitable ACE-2 reducing component include any compounds or compositions which are capable of reducing ACE-2 expression levels and availability in the mammal. Of known ingredients, the inventors of the present disclosure have discovered that L-carnitine tartrate is an effective ingredient for reducing ACE-2 in a mammal.

The ACE-2 reducing component may be administered occasionally or on a regular schedule. Depending on the method of delivery, ACE-2 reducing component may be administered once per month, twice per month, once per week, twice per week, three times per week, four times per week, five times per week, six times per week or on a daily basis. regularly, for best results, For oral administration, ACE-2 reducing component is administer in a regular basis, such as once or twice a day, or every other day. Dosing will depend on the particular ACE-2 reducing component.

ACE-2 reducing component may be administered via an oral dose in the form of a liquid or solid, in a capsule or as a tablet or other suitable oral dosing form. Other methods may also be used, including, intranasal by inhalation, intravenous, intramuscular, intragastric, transdermal and the like.

In one embodiment of the disclosure, the ACE-2 reducing component , is administered orally, typically in the form of a liquid or a solid preparation, such as a tablet, capsule, powder, or a gel or a paste. The dosage form is selected in view of the route of administration. Examples of oral preparations include powders, granules, tablets, capsules, pills, enteric coated preparations, liquids for internal use, suspensions, emulsions and syrups. To prepare these dosage forms, auxiliary agents commonly used in the field of pharmaceutical manufacturing technology, such as excipients, binders, carriers, antiseptics, antioxidants, disintegrators, lubricants, and flavoring agents, can be used as necessary. Auxiliary agents may be used alone or in combination of two or more of the auxiliary agents. From the above, a tablet, powder or solution is preferred. The ACE-2 reducing component may also be provided as an effervescent tablet or powder or the like for preparing a liquid preparation.

In another embodiment of the invention, the ACE-2 reducing component is administered as part of a food and drink as a nutritional supplement. The food or drink is a functional food or drink. Preferred drinks are energy drinks or energy shots. It may be used in conjunction with common additives for food products, such as sweeteners, seasonings, antiseptics, preservatives, germicides and antioxidants. In a preferred embodiment, the drink or food comprises the desired dose for a single administration.

Typically, the composition comprises at least one carrier and is in the form of a tablet, or capsule . The ACE-2 reducing component may also be used in a combination with at least one carrier. For example, the carrier is selected from cellulose or microcrystalline cellulose and cellulose derivatives like HPMC, HPC and ethylcellulose, starch and modified starch, Ca-carbonates and Ca-silicates, lactose, sugars or sugar alcohols and derivatives and edible wax. The carrier is generally not critical to the advantage of the regimen of the ACE-2 reducing component.

The nutritional supplement may further comprise acetyl L-carnitine, valeryl L-carnitine, isovaleryl L-carnitine, benzyl L-carnitine, L-leucyl L-carnitine, L-valyl L-carnitine, other L-amino acyl carnitines, salts of L-amino acyl L-carnitine, L-carnitine HCL, L-carnitine fumarate, propionyl L-carnitine, L-carnitine phosphate, acetyl L-carnitine L-aspartate, acetyl L-carnitine citrate, acetyl L-carnitine maleate, acetyl L-carnitine phosphate, acetyl L-carnitine fumarate, propionyl L-carnitine orotate, acetyl L-carnitine orotate, butyryl L-carnitine orotate, propionyl L-carnitine fumarate, L-carnitine oxalate, L-carnitine sulfate, GPLC glycine propionyl L-carnitine or a mixture thereof. Of the L-carnitine compounds, L-carnitine tartrate is generally used for its known flowability, since it is less hydroscopic that other L-carnitine compounds. The ACE-2 reducing component is L-carnitine tartrate.

L-carnitine may be administered via an oral dose in the form of a liquid or solid, in a capsule or as a tablet or other suitable oral dosing form. Other methods may also be used, including, intranasal by inhalation, intravenous, intramuscular, intragastric, transdermal and the like.

The carnitine, in particular L-carnitine, can be administered occasionally or on a regular schedule. Depending on the method of delivery, carnitine may be administered once per month, twice per month, once per week, twice per week, three times per week, four times per week, five times per week, six times per week or on a daily basis. For oral administration, carnitine is administer in a regular basis, such as once or twice a day, or every other day. For instance, the carnitine nutritional supplement may be administered to the mammal at least every one to three days.

In one particular embodiment, the carnitine nutritional supplement is administered daily. The dosage can be from about 5 to 10,000 milligrams per day, such as from about 50 to about 7,000 milligrams per day, such as from about 100 milligrams to about 5000 milligrams per day. The dosage, for instance, can be greater than about 100 milligrams per day, such as greater than about 150 milligrams per day, such as greater than about 200 milligrams per day, such as greater than about 250 milligrams per day. Typically, in humans, the L-carnitine is administered in an amount of about 250mg to 5000 mg per day. More typically, L-carnitine is administered in the range of 500mg to 4000mg per day and generally in amount between 1000mg and 2500mg per/day.

In addition to dose based on a daily dose L-carnitine can be administered on a per kilogram basis. Based on body mass, the dosage can be from about 1 milligram per kilogram of body weight per day to about 500 milligrams per kilogram body weight per day, depending on the mammal. For example, the dosage may be from about 5 milligrams per kilogram body weight per day to about 50 milligrams per kilogram body weight per day in humans. For other mammals, the carnitine dosage can be greater than 50mg per kilogram body weight per day. For the purposes of humans, carnitine supplementation is based on a 60 kg as the average human weight.

Dosing of L-carnitine may be done once a day, twice a day, by dividing the daily does into half, or may be dosed up to four times per day or more. Generally dosing is either once a day or twice a day.

The L-carnitine nutritional supplement can be administered to the mammal in any suitable form using any suitable administration route. For example, the L-carnitine nutritional supplement can be administered orally alone, in combination with a food composition, as part of a food composition or as part of a multicomponent nutritional supplement or nutraceutical composition, wherein the other supplement or composition contains other nutraceutical active ingredients. The L-carnitine nutritional supplement may also be part of a dietary nutritional supplement or as a nutraceutical composition. The L-carnitine nutritional supplement, in one embodiment, may only contain L-carnitine to receive the benefits and advantages of the present disclosure without containing other dietary nutritional supplements, such as amino acid supplements.

L-carnitine has beneficial effects on the mitochondria function. L-carnitine shuttles long-chain fatty acids inside the mitochondria by forming a long chain acetylcarnitine ester. The complex is then transported into the mitochondrial matrix by carnitine palmitoyltransferase I (CPT I) and carnitine palmitoyltransferase II (CPT II). The fatty acids are then broken down through the process of β-oxidation to deliver the 2-carbon molecules to the Krebs cycle, leading to the generation of energy under the form of adenosine triphosphate (ATP). In addition, by binding an acetyl group, L-carnitine can maintain the levels of Acetyl-CoA and coenzyme A, playing its buffering role. L-carnitine reduces oxidative stress and inflammation and by reducing the oxidative stress and inflammation, the ACE-2 levels may also be reduced. L- carnitine has been shown to also help assist in down regulating serine proteases TMPRSS2 and Furin, two other proteins (peptidases) which may provide entry and processing into host cells by infectious agents in the rodent model.

When the L-carnitine nutritional supplement is combined with a food or beverage composition, the food or beverage composition may comprise any suitable composition for consumption by the mammal. Such compositions include complete foods or beverages intended to supply the necessary dietary requirements for mammal or food supplements such as treats and snacks. The food composition may comprise pellets, a drink, a bar, a prepared food contained in a can, a dairy food product, or any other functional food composition The food composition may also comprise any form of a nutritional supplement such as a pill, soft gel, gummy figurine, wafer, or the like.

The food composition ingested by the mammal in addition to the L-carnitine nutritional supplement may also be rich in L-carnitine. The L-carnitine nutritional supplement of the present disclosure, for instance, is intended to provide additional L-carnitine in addition to normal amounts of L-carnitine contained in a standard diet and/or the amount of L-carnitine produced by the body.

For best effect, it is preferable that the carnitine supplementation be used prior to an infection with an infectious agent. Generally, carnitine should be taken at least one day before a potential contact with an infectious agent, typically at least three days before and more typically at least one week before potential contact with a infectious agent. Desirably, the carnitine supplementation should be two or more weeks before potential contact with the infectious agent, for example 3 weeks, 4 weeks, 5 weeks, 6 weeks, 7 weeks or 8 weeks or longer. The carnitine should be administered on at least a daily basis

In accordance with the present disclosure, the L-carnitine supplementation is effective against various infectious agent which binds a receptor of an ACE-2, by reducing (down regulate) the ACE-2 in the body to near basal levels, when the mammal is subjected to stressors that will increase ACE-2 levels. These include virus, such as coronavirus, including but not limited to SARS-COV-2 and the related disease COVID-19.

The ACE-2 reducing component may be used alone to treat diseases associated with SARS-COV-2 or may be used in combination with one or more other

Carnitine supplementation may also be effective in avians as well to prevent respiratory viral diseases.

Nonetheless, certain embodiments of the present disclosure may be better understood according to the following examples, which are intended to be non-limiting and exemplary in nature.

### EXAMPLE 1

To show the effects of carnitine supplementation has on the ACE-2 the following experiments were run in rodents.

Fifty-six (56) Male Wistar rats that were 8-week old were obtained. The rats were reared at the temperature of 22 ± 2°C, the humidity of 55 ± 5%, and with a 12-hrs light-12-hrs dark cycle. The animals were fed a regular diet shown in Table 1 and water ad libitum. All procedures involving rats were conducted in strict compliance with the relevant laws, the Animal Welfare Act, Public Health Services Policy, and guidelines established by the Institutional Animal Care and Use Committee of the Institute of the contracted University which completed the study.

**Table 1 Ingredient and Nutrient Composition of Rat Diet**

| Ingredient | | Percentage on weight basis |
|---|---|---|
| | Maize | 22.00 |
| | Wheat | 15.00 |
| | Soybean meal, 48% CP | 15.10 |
| | Sunflower meal, 30% CP | 15.00 |
| | Wheat bran | 9.00 |
| | Molasses | 9.00 |
| | Limestone | 2.00 |
| | Salt | 0.80 |
| | DL-Methionine | 0.80 |
| | Dicalcium phosphate | 1.00 |
| | Vitamin and mineral premix¹ | 0.30 |

| | | |
|---|---|---|
| ¹The vitamin-mineral premix provides the following (per kg): all-trans-retinyl acetate, 1.8 mg; cholecalciferol, 0.025 mg; all-rac-a-tocopherol acetate, 12,5 mg; menadione (menadione sodium bisulfate), 1.1 mg; riboflavin, 4.4 mg; thiamine (thiamine mononitrate), 1.1 mg; vitamin B-6, 2.2 mg; niacin, 35 mg; Ca-pantothenate, 10 mg; vitamin B-12, 0.02 mg; folic acid, 0.55 mg; d-biotin, 0.1 mg. manganese (from manganese oxide), 40 mg; iron (from iron sulfate), 12.5 mg; zinc (from zinc oxide), 25 mg; copper (from copper sulfate), 3.5 mg; iodine (from potassium iodide), 0.3 mg; selenium (from sodium selenite), 0.15 mg; choline chloride, 175 mg | | |

Analysis of the Nutrient Composition showed that the composition contains Crude protein 24.12%, Ether extract 2.43%, Crude cellulose 7.09%, Ash 8.25%, Calcium 1.28% and Phosphorous 0.85 %, all percent by weight.

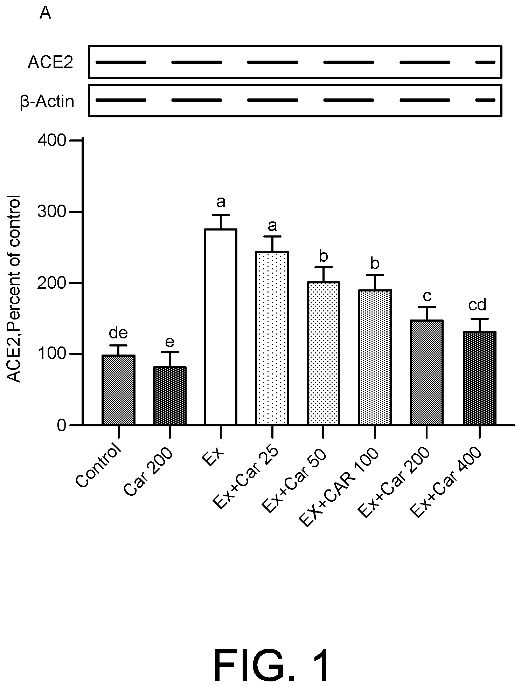

After a week of the adaptation period, the rats were divided into eight equal groups containing seven rats each. The initial body weights of the rats were similar in all the groups (214.53 grams ± 4.67; P > 0.05). The eight groups are as shown in FIGURES 1-4:
1) control with no exercise or carnitine supplementation;
2) administered 200 mg/kg of L-carnitine tartrate supplementation with no exercise (Car 200);
3) exercise with no L-carnitine supplementation (Ex);
4) exercise with 25 mg/kg carnitine supplementation (Ex+Car 25);
5) exercise with 50 mg/kg carnitine supplementation (Ex+Car 50);
6) exercise with 100 mg/kg carnitine supplementation (Ex+Car 100);
7) exercise with 200 mg/kg carnitine supplementation (Ex+Car 200); and
8) exercise with 400 mg/kg carnitine supplementation (Ex+Car 400).

L-carnitine was fed to the rats in Groups 2, and Groups 4-8 via oral gavage.

Groups 1 and 2 were not subjected to an exercise routine. Groups 3-8 were subjected to an exercise regimen consisting of a week training period with an incremental exercise capacity described below. After the week training period the exercise regimen followed was each rat in Groups 3-8 were subjected to 6 weeks of running at 25 m/min during 45 min every day. L-carnitine supplementation was administered (mg per body weight) 0, 25, 50, 100, 200 and 400 mg/kg, for each group as the case may be for the entire 6-week exercise period for the exercised groups. These correspond to 0, 250, 500, 1000, 2000 and 4000 mg per day for humans, respectively. Also L-carnitine supplementation was provided to Group 2 during the entire 6-week period as well at a level of 200 mg/kg.

The exercised rats were subjected to treadmill exercise on a motorized rodent treadmill (Commat Limited, Ankara, Turkey). The treadmill contained a stimulus grid at the back end of the treadmill giving an electric shock when the animal placed its paw on the grid. The apparatus had a 5-lane animal exerciser utilizing a single belt unit divided with walls suspended over the tread surface. To eliminate the diurnal variations, all exercise tests were applied during the same time of the day. The week of training period was provided as pre-training practice for the animals to get familiar with the treadmill equipment and handling. In doing so, the rats in the exercise training groups were accustomed by treadmill exercise over 5 days such that: (i) 1st day, 10 m/min for 10 min, (ii) 2nd day, 20 m/min for 10 min, (iii) 3rd day, 25 m/min for 10 min, (iv) 4th day, 25 m/min for 20 min and (v) 5th day, 25 m/min for 30 min. After the training period, the rats in treadmill exercise groups ran on the treadmill 25 m/min for 45 min/ per day and five days per week for 6 weeks according to the protocol described by Sahin et al. Capsaicinoids Improve the Consequences of Physical Activity. Toxicol Rep. 2018;5:598-607, which is hereby incorporated by reference.

At the end of each exercise session, exhaustion time and average distance run were recorded. Endurance capacity was measured using treadmill running to fatigue after the end of each training session by compelling the rats to run on a motorized defined as the inability of the rat to maintain an appropriate pace despite continuous hand prodding for 1 min. After this application, the rats were removed from the treadmill.

The exercise regimen was used to induce an inflammation and oxidative stress conditions. L-carnitine supplementation led to an increase in the anti-oxidant capacity as demonstrated by an increase in superoxide dismutase, catalase, and glutathione peroxidase in the rats and a further decrease in malondialdehyde. The results of malondialdehyde, superoxide dismutase, catalase, and glutathione peroxidase for each rat group are shown in TABLE 2.

**Table 2 Antioxidant Capacity**

| Items | Groups | | | | | | | |
|---|---|---|---|---|---|---|---|---|
| | Control | Car200 | Exercise (Ex) | Ex+Car25 | Ex+Car50 | Ex+Car100 | Ex+Car200 | Ex+Car400 |
| MDA, µmol/L | 0.873±0.017^{a} | 0.693±0.021^{b} | 0.768±0.015^{b} | 0.599±0.021^{c} | 0.549±0.014^{cd} | 0.553±0.018^{cd} | 0.489±0.019^{de} | 0.419±0.009^{e} |
| SOD, U/mg protein | 83.88±2.21^{e} | 95.87±2.47^{cd} | 85.21±2.99^{de} | 96.49±0.78^{cd} | 102.89±2.69^{bc} | 109.68±2.49^{ab} | 114.56±2.70^{a} | 120.16±3.58^{a} |
| CAT, U/mg protein | 135.51±2.61^{e} | 147.19±2.01^{de} | 138.47±3.69^{e} | 144.55±3.21^{de} | 152.72±2.50^{cd} | 162.11±1.97^{bc} | 170.77±2.57^{b} | 186.10±2.50^{a} |
| GSHPx, U/mg protein | 36.87±1.27^{f} | 48.74±1.11^{d} | 42.56±1.43^{e} | 49.75±1.08^{d} | 55.91±0.85^{c} | 59.57±0.68^{bc} | 62.58±1.03^{b} | 69.06±1.53^{a} |

| | | | | | | | | |
|---|---|---|---|---|---|---|---|---|
| MDA: malondialdehyde; SOD: superoxide dismutase; CAT: catalase; GSH-Px: glutathione peroxidase. Statistical comparisons are indicated with different superscript (a-f) in the same row (P < 0.05; *ANOVA and Turkey's *post-hoc test*). | | | | | | | | |

In additions, inflammation and oxidative stress conditions were used to demonstrate the effects of L-carnitine supplementation has on reducing ACE-2 in rats in an inflammation state. After the 6-week exercise regimen was completed, muscle, lung, plasma and liver tissue samples were taken from these rats. The ACE-2 analysis of the tissue samples are subjected to the following test.

Exercise was used with the rats to induce an inflammation condition to demonstrate the effects of L-carnitine supplementation has on reducing ACE-2 upon an increase as a result of the exercise-induced inflammation.

To determine the protein accumulation in the liver, muscle and lung samples, a Western Blotting test was used. Each of the tissue samples of the same group were pooled, homogenized, and sonicated in lysis buffer supplemented with 1X protease and phosphatase inhibitor cocktail (#5872, Cell Signaling). Total protein content was detected with Qubit 2.0 Fluorometer according to the manufacturer's protocol (Invitrogen, Life Technologies Corporation, Carlsbad, CA, USA). Equal amounts of protein (20 µg) were size-fractionated using any-kD Mini-Protean TGX gel electrophoresis and then transferred to a nitrocellulose membrane using the Trans-Blot Turbo Transfer System (Bio-Rad, Life Sciences Research). Thereafter, membranes were blocked in 5% nonfat milk in Tris-buffered saline (TBS) containing 0.1% Tween (TBS-T; blocking solution) for 1 h at room temperature, washed in TBS-T, and incubated overnight with ACE-2 (Abcam, Cambridge, UK). The following day, membranes were washed with TBS-T and then incubated with horseradish peroxidase-conjugated goat anti-rabbit (Abcam, Cambridge, UK) or goat anti-mouse (Abcam, Cambridge, UK) secondary antibody (diluted 1:1000) for 1 h. Individual blots were performed at least three times. Protein loading was controlled by stripping and re-probing the nitrocellulose membrane with an anti-β-actin antibody (Abcam, Cambridge, UK). Protein levels were analyzed densitometrically using an image analysis system (Image J; National Institute of Health, Bethesda, MD, USA), corrected with values determined on β-actin blots and expressed as relative values compared with the control group.

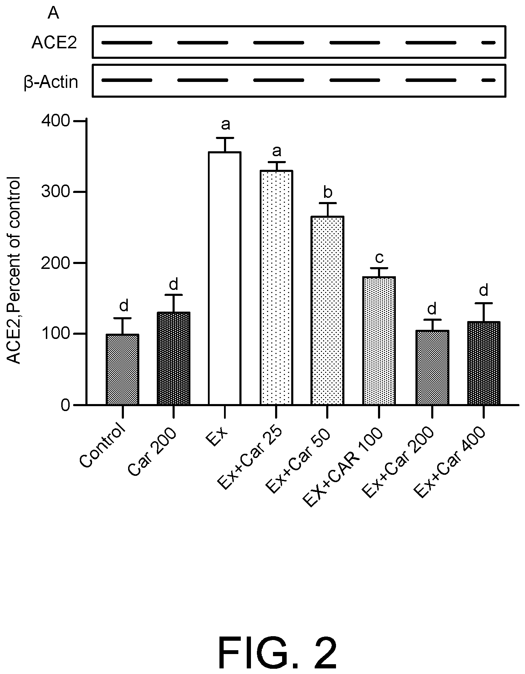

The results of the western blotting test are show in FIGURES 1 and 2. FIGURE 1 shows the results for liver samples and FIGURE 2 shows results for the muscle samples. The I-bar represents the standard error in each group and the letters above the bars show which groups are significantly different from the control group (P < 0.05, statistically different compared with the control group). When bars are labeled with different letters, it means that these comparisons are significant.

To determine the mRNA expression levels of ACE-2 in the liver, lung and muscle samples a total RNA extraction and specific mRNA quantification was conducted by reverse transcription-polymerase chain reaction (RT-qPCR). Gene expressions were done by quantitative polymerase chain reaction (qPCR). Total RNA was extracted from frozen tissues samples using a RNeasy 96 Universal kit (Qiagen, Tokyo, Japan) according to the manufacturer's instructions. For this purpose, the sample was homogenized using a Tissue Ruptor (Qiagen) homogenizer attached with a probe in 600 µl of 1:100 beta-mercaptoethanol in RLT buffer, and centrifuged for 3 min. After mixing the supernatant with 1 volume of 70% ethanol, samples were further processed for total RNA using RNeasy Total RNA Mini Kit (Qiagen) according to the manufacturer's instructions. The RNA samples were quantified using NanoDrop (MaestroGen, Las Vegas, NV, USA). cDNA was synthesized from 500 ng of total RNA using the High Capacity Reverse Transcription cDNA kit containing random primers (Qiagen, Valencia, CA, USA). Real-time quantitative RT-PCR was done on cDNA aliquots with a SYBR Green PCR Master Mix (Catalog no. 330620, Qiagen) to quantitatively evaluate the gene expression of ACE-1, ACE-2, TMPRRS-2, Furin on a Rotor-Gene Q machine (Qiagen, Hilden, Germany). Glyceraldehyde-3-phosphate dehydrogenase (GAPDH) gene was used as the internal control. The primers used for the amplification are listed in Table 3. Reactions were performed in triplicates with 2 µl primer pair, 5 µl SYBR green master mix, 1 µl RNA-free water and 2 µl cDNA templates. PCR was done with the following conditions: initial denaturation at 95 °C for 10 minutes followed by 40 cycles denaturation at 95 °C for 10 seconds, annealing at 60 °C and extension together at 60 °C at 45 °C. Each PCR reaction was made in triplicate and the mean Ct value was used for statistical analysis. mRNA expression was standardized for GAPDH expression levels and then normalized to the control group.

**Table 3. Forward and reverse primer sequences of genes used for real-time polymerase chain reaction.**

| Gene | Forward primer (5'→3') | Reverse primer (5'→3') |
|---|---|---|
| ACE-1 | AGCATCACCAAGGAGAACTA | ACTGGAACTGGATGATGAAG |
| ACE-2 | GCTCCTGCTGGCTCCTTCTCA | GCCGCAGCCTCGTTCATCTT |
| TMPRRS-2 | CACCTGCCATCCACATACAG | CCAGAACTTCCAAAGCAAGC |
| FURIN | ACTAACACTGTGCCCTGGTG GAG | ACCCTGGACAGGTAGGTTGGGTA |
| GAPDH | GTGGTGAAGCAGGCATCTG | GTGGTGAAGCAGGCATCTG |

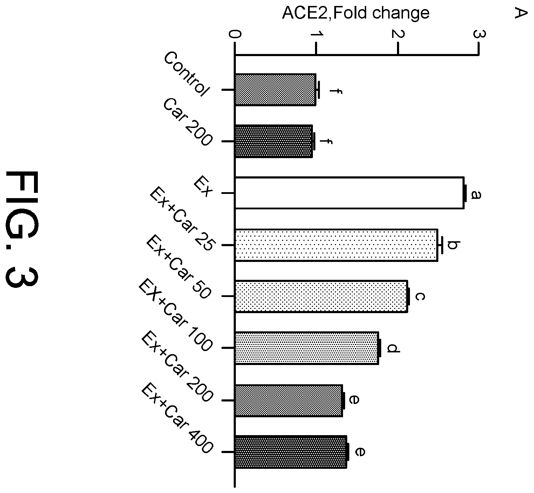

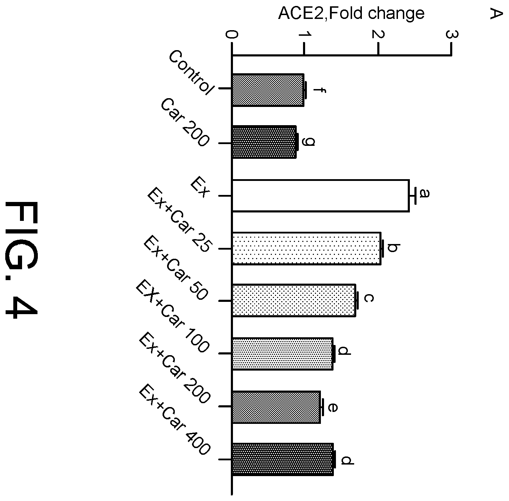

The results of the mRNA quantification test are show as fold change in FIGURES 3 and 4. FIGURE 3 shows the results for liver samples and FIGURE 4 shows results for the muscle samples. The I-bar represents the standard error in each group and the letters above the bars show which groups are significantly different from the control group (P < 0.05, statistically different compared with the control group). When bars are labeled with different letters, it means that these comparisons are significant.

The testing demonstrate, as is clearly shown in FIGURES 1-4 that L-carnitine supplementation in exercising rats showed significant and dose dependent decrease in the ACE-2 to reach a control levels (without exercise). These effects are seen only with exercise which was used to induce an inflammation condition in the rats. This is very important as the ACE-2 has physiological roles, and their complete depletion can introduce serious safety effects:

ACE-2 regulates blood pressure (beneficial) in addition to its role as a SARS-CoV- receptor which will bind with an infectious agent having a SARS-CoV (detrimental).

L-carnitine supplementation to the diet of the rats resulted in a decrease in the ACE-2 levels only under exercise condition (to induce inflammation) means that potential safety concerns on hypertension is alleviated. Indeed the results indicate that without exercise (inflammation) L-carnitine does not affect these receptors. This is supported by what is known with respect to the beneficial effects of I-carnitine on both hypertension and diabetes.

Some COVID-19 patients, including patients with high inflammation states, such as those with uncontrolled hypertension and poorly controlled hypertension, are more susceptible to a severe COVID-19 infection. In addition, other groups, such as pregnant women, elderly, metabolic syndrome patients (hypertension, and diabetic) have a significant decrease in their plasma L-carnitine. Knowing that L-carnitine can decrease ACE-2 in the body of a mammal in an inflammation state, it is concluded that L-carnitine will be effective in reducing the likelihood that a patient in a high inflammation condition will be more susceptible to a corona virus infection by reducing the number of available receptors in the body of the mammal. This can be done without adversely affecting the beneficial effects of ACE-2.

### EXAMPLE 2

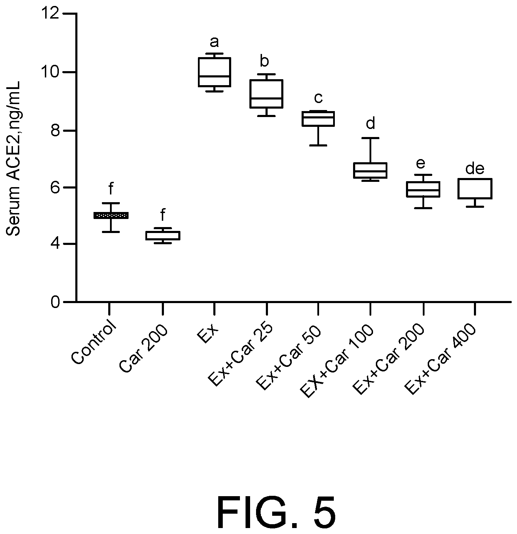

In this example, the ACE-2 levels in plasma serum of the rats tested in Example 1 were detected using a commercially available assay Rat Kit (Rat ACE / CD143 (Competitive EIA) ELISA Kit - LS-F27855), available from LSBio, Seattle, WA, USA. All of the manufactures instructions for the Rat Kit were followed to determine the ACE-1, ACE-2, ACE-1/ACE-2 ratio and TMPRSS2 levels in the plasma serum. ACE-2 sensitivity was 0.312 ng/mL and Intra-Assay: CV<5.3% / Inter-Assay: CV<8.2%. The results of the ELISA kit testing is shown in FIGURES 5A, 5B, 5C and 5D. As can be seen in FIGURE 5A, the carnitine supplementation is effective controlling the ACE-1 in rats subject to an inflammation state (exercised group) to near normal levels as compared to the control. FIGURE 5B shows the reduction in the ACE-2 levels in the plasma serum of the exercised group. FIGURE 5C shows the ratio of the ACE-1 to ACE-2 in the plasma serum closer to the control levels as compared to the exercised group. Finally, FIGURE 5D shows the effect of controlling TMPRSS2 in rat plasma serum with carnitine supplementation. This suggests that L-carnitine is effective for treating a mammal which is infected with an infectious agents that binds to the ACE-2 receptor, such as SARS CoV viruses and treating the related disease associated with SARS CoV, such as COVID-19.

### EXAMPLE 3

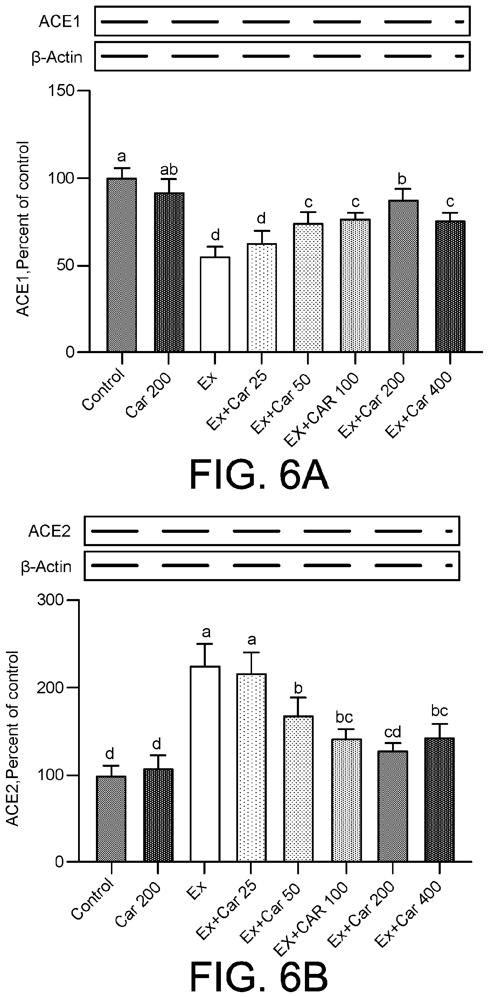

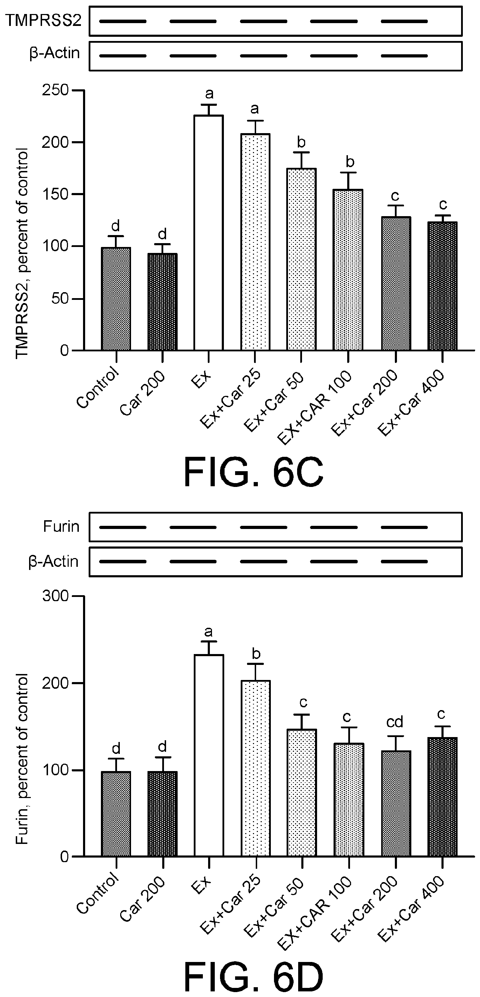

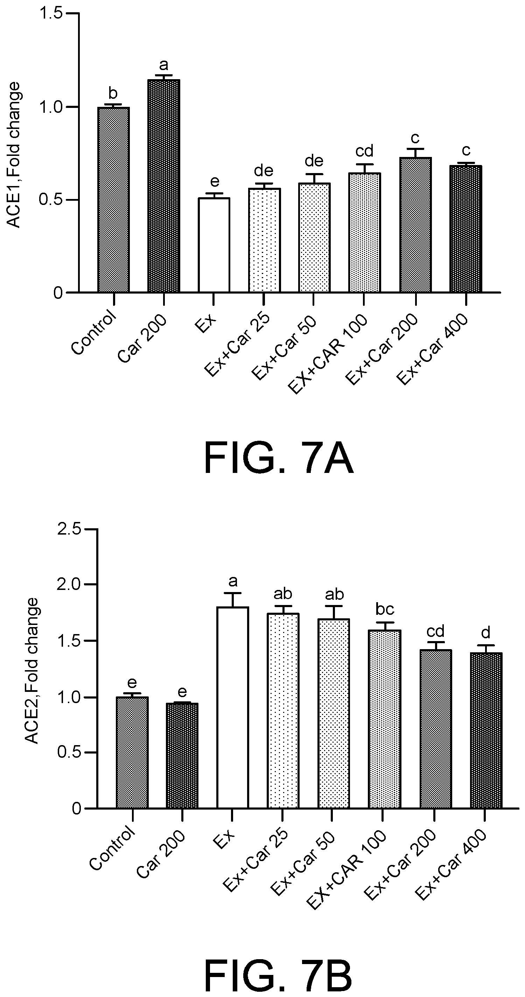

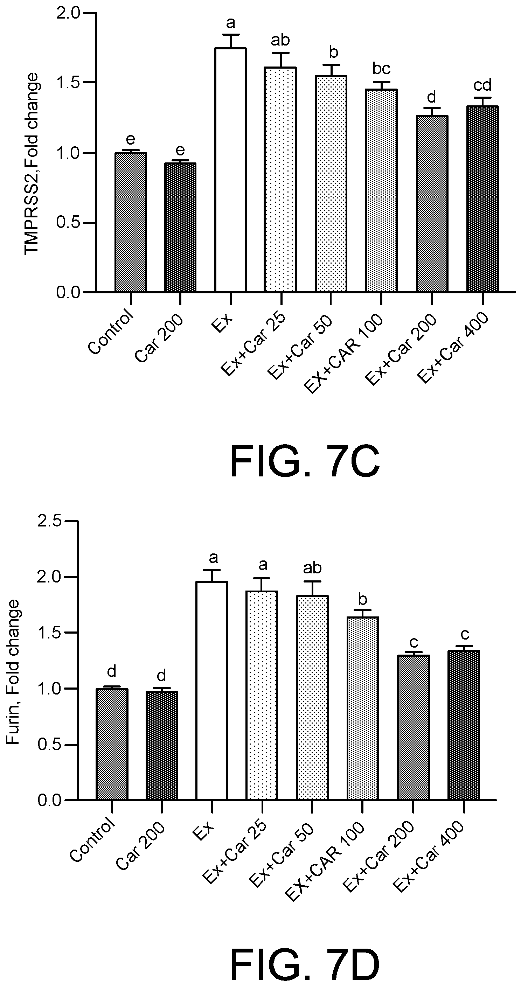

In this example, the effects of carnitine supplementation was measured in lung tissue for ACE-1, ACE-2, TMPRSS2 and Furin protein expression levels taken form the rats tested in Example 1 in the lung tissue samples, as described above. The intensity of the bands shown was quantified by densitometric analysis. The bar represents the standard error of the mean. Blots were repeated at least 3 times (n=3) and a representative blot is shown. β-actin was included to ensure equal protein loading. (a-d); P < 0.05, statistically different compared with the control group. The results are shown in FIGURE 6A for ACE-1 expression. ACE-2 results are shown if FIGURE 6B. TMPRSS2 results are shown in FIGURE 6C and Furin results are shown in FIGURE 6D. The fold change, which is the expression relative to that in untreated cells, in the ACE-1, ACE-2, TMPRSS2 and Furin protein in Lung Samples is shown in FIGURES 7A,7B, 7C and 7D, respectively.

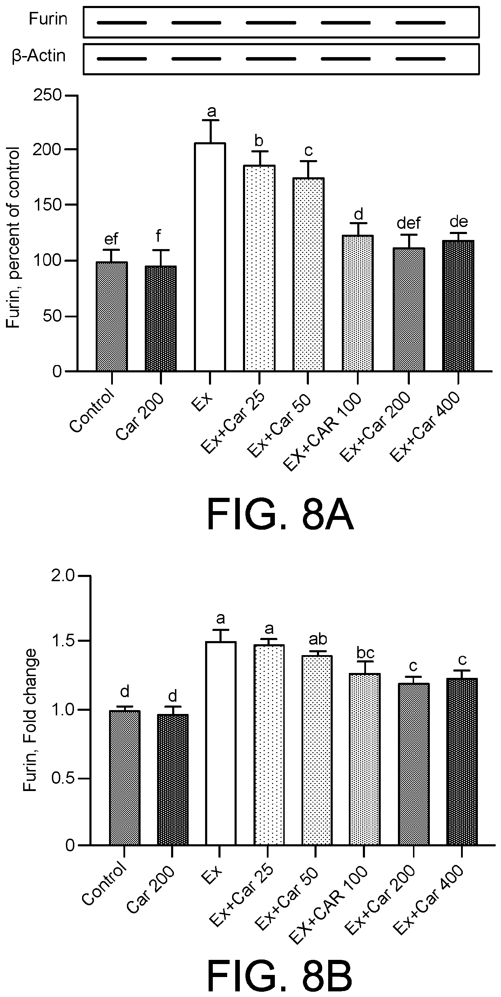

In addition Furin protein expression levels in the liver of the rats tested in Example 1 was also measured, as described above in Example 1. The results of the Furin protein express are shown in FIGURE 8A and the fold change in RNA expression is shown in FIGURE 8B. Each of the eight groups is the same as those in Example 1.

### EXAMPLE 4

A study was run examined the long-term effects of L-Carnitine Tartrate, on resting long-term systemic immune mediated pro-inflammatory markers along with receptors known to play a role in virus reproduction.

A human study was run with a total of 80 healthy male and female subjects ranging from 21 to 65 years of age, who were active (i.e. 30 minutes per day of moderate activity, 3 days per week) are randomized into two groups. One group was supplemented with 2 g daily of L-carnitine tartrate and one group was supplemented with a placebo matching control for a period of 5 weeks. Seventy-three subjects completed the trial and biomarker data were analyzed from the sera of the 73 completers. Sera were collected for analysis of C-Reactive Protein (CRP), ACE 1, ACE 2, TMPRSS 2, the Tumor Necrosis Factor alpha (TNF-alpha), and Furin at baseline in the beginning of the trial, at week 5 and 48 hr after an exercise challenge as described (Clinicaltrials.gov NCT04420377). The exercise challenge serves as surrogate to the inflammation stimulus.

To obtain human sera, venous blood was extracted by venipuncture of the antecubital vein using a 21-gauge syringe and collected into a 10mL EDTA vacutainer tube (BD Vacutainer^{®}, Becton, Dickinson and Company, Franklin Lakes, NJ) by a certified phlebotomist. Blood samples were centrifuged at 2500 rpm for 10 minutes at 4°C. Resulting serum samples were then aliquoted and stored at -80°C until further analysis. Human ACE-1, ACE-2, TMPRSS2, Furin, CRP and TNF-alpha protein levels were assessed using commercially available ELISA kits, according the manufacturer recommendations, ACE-1 (R&D systems Inc., Minneapolis, MN, USA), ACE-2 (RayBiotech inc., Peachtree Corners, GA), TMPRSS2 (Novus Biologicals, Littleton, CO), CRP (R&D Systems Inc., Minneapolis, MN), TNF-alpha (Novus Biologicals, Littleton, CO), and Furin (Sigma Aldrich, St. Louis, MO).

To assess the effects of L-carnitine on the different biomarkers, sera from 73 completers of the human trial were collected at baseline, after 5 weeks of supplementation and 48 hours after exercise challenge.

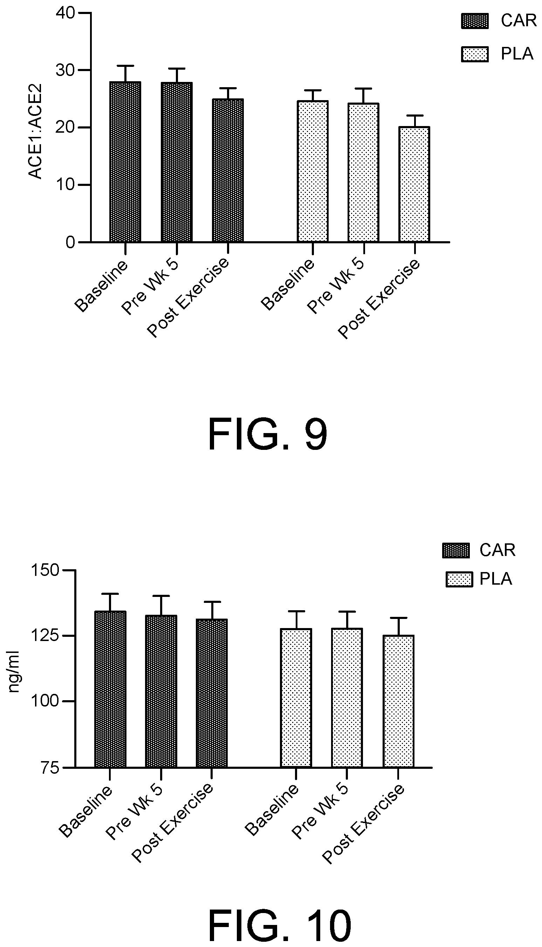

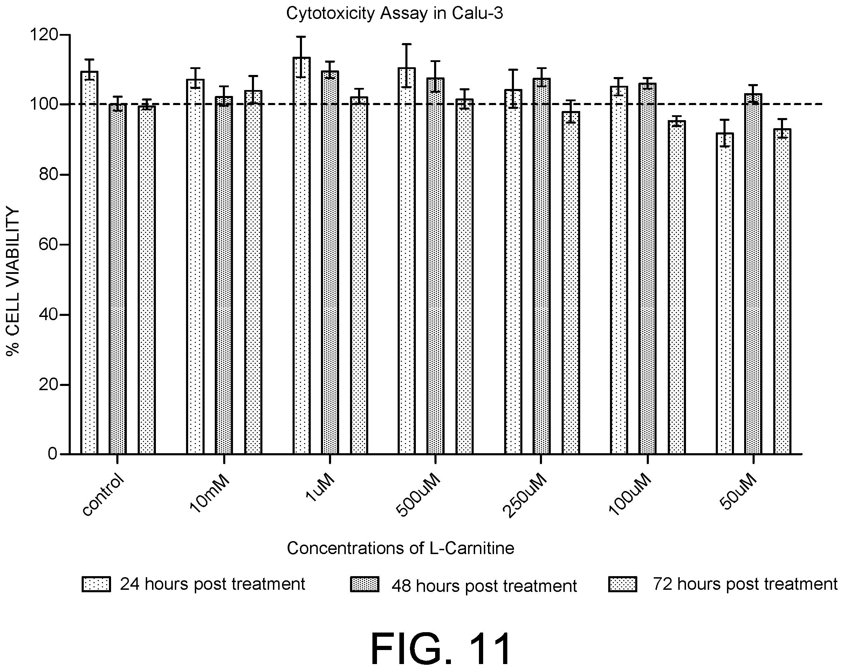

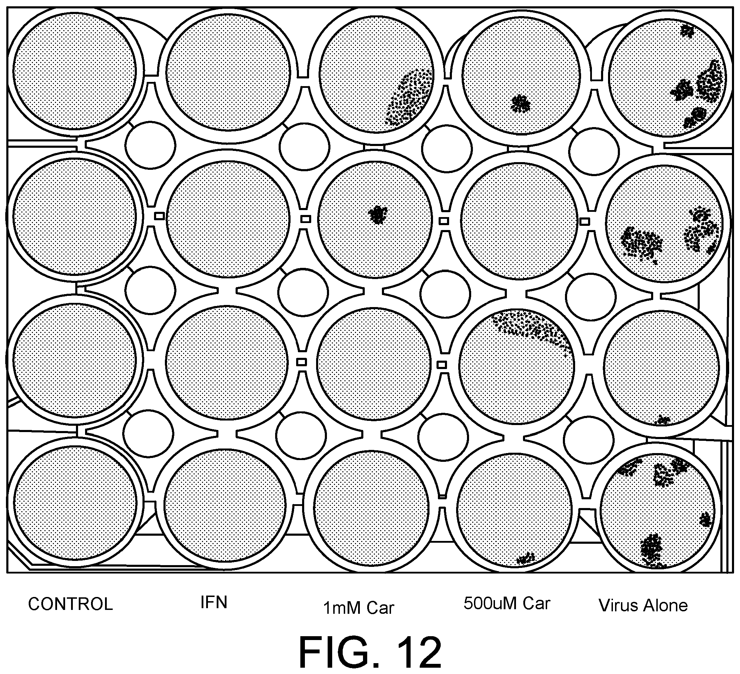

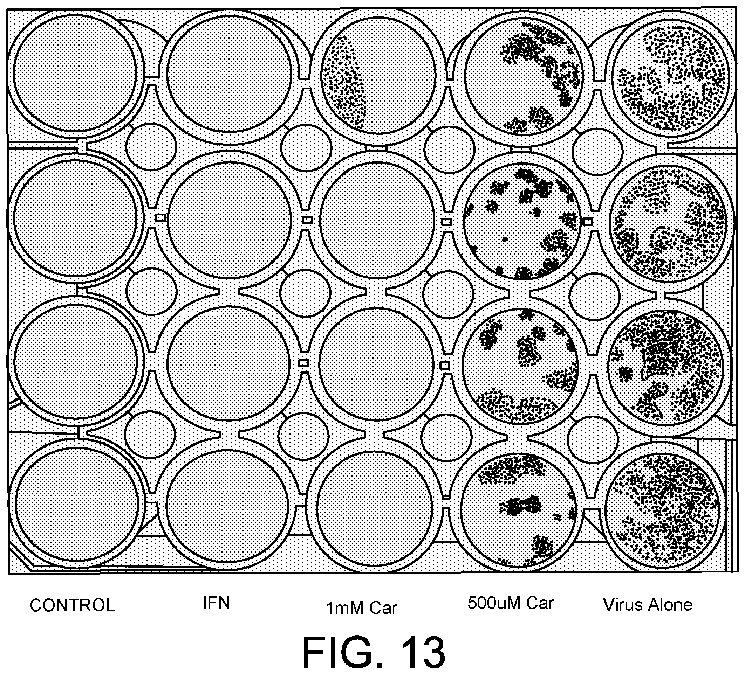

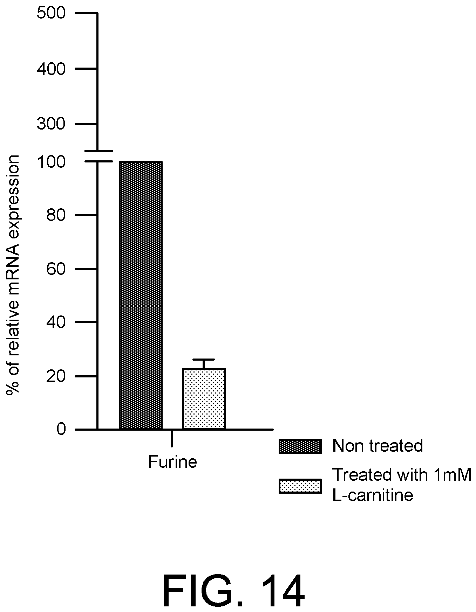

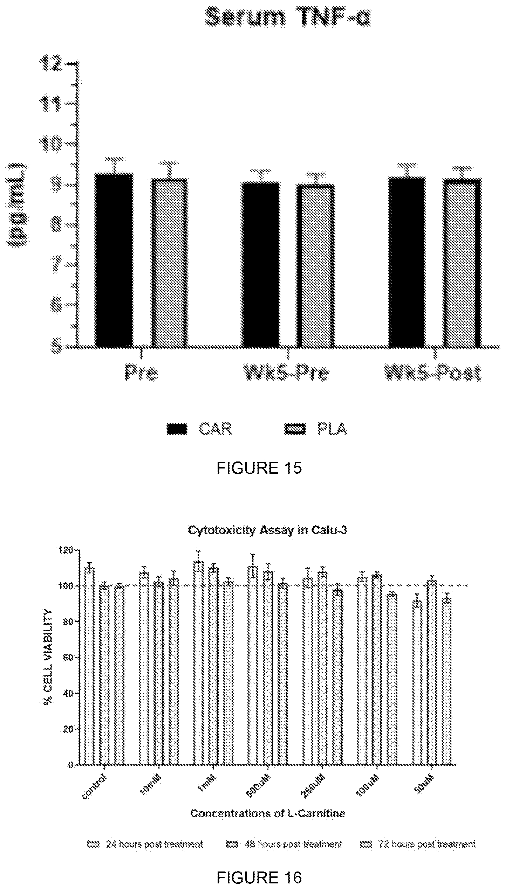

FIGURE 9-15 show the effect of carnitine supplementation on ACE-1, ACE-2, Furin, TMPRSS2, the ratio of ACE-1/ACE-2, CRP and TNF-alpha, respectively. The error bars have the following meaning: a=different than Pre (p<0.05), b= different than Week 5-Pre (p<0.05), c= different than Week 5-post, and d= different between groups (p<0.05). FIGURE 9 shows no significant effect of L-carnitine on ACE-1 serum levels before or after exercise challenge (p>0.05), suggesting that inflammation resulting from exercise did not affect ACE-1, the pro-inflammatory marker. However, a significant decrease was seen for ACE-2 (FIGURE 10), TMPRSS2 (FIGURE 11) and Furin (FIGURE 12). Post hoc analysis indicated that ACE-2, TMPRSS2 and Furin rose at 48 hours after the exercise challenge in the placebo, but not in the L-carnitine group compared to baseline and at week 5. The difference between groups was significant (FIGURE 12). No changes occurred in the ACE-1/ACE-2 ratio (as is shown in FIGURE 13). There was a significant group by time interaction for serum CRP and post-hoc analysis revealed that CRP levels was significantly lower from baseline after 5 weeks of L-carnitine supplementation as compared to placebo group (FIGURE 14). The between group changes was also significant. No change occurred in TNF-alpha levels at any time point, nor was there an impact of supplementation FIGURE 15.

Changes between time points were measured using t-tests. The alpha level was set *a priori* at p<0.05.

### Results

Dependent variables were scrutinized using a two-way mixed analysis of variance (ANOVA) with condition as the "between-group" factor (CAR = carnitine & PLA = placebo), time as the "within-group" factor (Pre, Wk5-Pre, Wk5-Post), and subjects as a random factor. For ANOVA procedures, homogeneity of variances and covariances were confirmed by Levene's test and Box's M test, respectively. Additionally, Mauchly's test of sphericity was used to test the assumption of sphericity for two-way interactions. For all analysis, the alpha level was set a priori at p<0.05. Data are presented as mean ± standard error unless otherwise stated.

CRP: As is shown in FIGURE 14 , which shows the plasma CRP Concentration (mg/L), it can be seen that the CRP was significantly lower in Group A than Group B at 48hr-Post (p<0.005, Mean diff = -0.55 mg/L)

ACE-2: As is shown in FIGURE 10, which shows the Plasma ACE-2 Concentration, it can be seen that there was a significant decrease in ACE-2 of 8% in-group A (p 0.046) from baseline compared to Wk5 (p=0.046) and from pre to 48 hours post exercise at Wk5 (p=0.048). No significant changes occurred in Group B.

TNF-α: As is seen in FIGURE 15, which shows the TNF-α was lower at Wk5 compared to BL (p<0.01) in group A. Additionally, when comparing the relative percent change, Group A experienced a greater reduction in TNF-α from BL to Wk5 (p<0.05, -9% vs +4%).

The results are graphically shown in FIGURES 9-15. FIGURE 13 shows Plasma Angiotensin-Converting Enzyme 1 to Angiotensin-Converting Enzyme 2 Ratio based on the results shown in Example 4. FIGURE 9 shows Plasma Angiotensin-Converting Enzyme 1 (ACE-1). As can be seen, there were no significant interaction or main effect detected for ACE-1 (p>0.05).

This date further suggest that carnitine can maintain the ACE-1/ACE-2 ratio constant a slight decrease. This implies that carnitine may provide a way to avoid the cytokine storm, which happens when the ACE-1/ACE-2 ratio increases.

As can be seen in Examples 1-4, the Exercise control group showed a significant increase in ACE-2, TMPRSS2 and Furin mRNA levels in all tissues (about 2 to 3-fold for ACE-2 and TMPRSS2 and 1.5-fold for Furin) and a significant decrease in ACE-1 mRNA levels about 1.5-fold (Figure 7A). L-carnitine supplementation led to a dose-dependent decrease in ACE-2, TMPRSS2 and Furin levels with a maximum effect at the 200 mg/kg dose. ACE-1 was significantly decreased with exercise and increased with L-carnitine supplementation as compared to the exercise control without reaching the control levels. The protein level assessment mimicked the mRNA effects and showed an increase of ACE-2, TMPRSS2 and Furin with exercise and a return to the baseline control with L-carnitine supplementation (FIGURES 3 and 4). ACE-1 protein levels showed similar pattern than the mRNA, with a decrease with exercise and an increase with the L-carnitine but significantly lower than the baseline control levels (FIGURE 6A). ACE-1/ACE-2 ratio was significantly decreased with exercise and L-carnitine supplementation increased this ratio but at a level of 60 % lower than the control, as shown in FIGURE 5C. This shows that a carnitine dietary supplementation could have a beneficial effect of reducing the chances of a mammal from becoming infected with the SARS-CoV-2 virus and having a COVID-19 infection, without leading to a spike of inflammation.

### EXAMPLE 5

In this example, human Calu-3 cells are treated with carnitine are checked for human ACE-2 gene expression.

### Calu-3 Cell Culture

Calu-3 cells were maintained in 50% DMEM (Dulbecco's Modified Eagle Medium) + 50% Ham's F12 media supplemented with 10% FBS (fetal bovine serum) and 1% penicillin/streptomycin. Vero E6 cells were maintained in DMEM media supplemented with 10% FBS and 1% penicillin/streptomycin.

### Calu-3 Cell Viability

Calu-3 seeded in a 96-well plate (30,000 cells) were stimulated with 50uM, 100uM, 250uM, 500 uM, 1mM and 10mM Carnitine alone. At 24h, 48h or 72h post stimulation, media was removed and cells were subjected to a standard MTT assay. The results of this testing is shown in FIGURE 16. L-carnitine did not show any cytotoxic properties on Calu-3 cells at any levels of L-carnitine.

### Evaluating surface expression protein in Calu-3 cells using flow cytometry

ACE-2 surface expression protein in Calu-3 cells was evaluated using FACS flow cytometry sorting. Cells were treated with L-carnitine for 24h as indicated above. Cells were detached with PBS/EDTA at room temperature. FBS-containing growth media was added to cells to inactivate TE. Cells were spun for 5 min at 850 RPMI. Supernatant was removed and cells were washed twice in flow buffer (PBS+0.2% FBS). Between 100,000-200,000 cells were stained for ACE-2 and BST2 (absent in Calu-3and used as a control) for 30 min on ice using goat anti-ACE-2 (R&D Systems; AF933) and PE-conjugated BST2 (BioLegend; Cat # 348406). Cells were washed once and stained with Alexa Fluor 647 donkey anti-goat for 30 min on ice. Cells were washed once, resuspended in PBS/EDTA and samples were acquired on a FACS LSR Fortessa cytometer (BD Bioscience). Data were analyzed using Flowjo (Version 10.1).

### Evaluating mRNA expression of ACE-2, Furin and TMPRSS2 in Calu-3 using RT real-time PCR

To quantify the human gene expression, Calu-3 seeded in a 12-well plate (220,000 cells) were stimulated with 100uM, 500 uM and 1000 uM Carnitine alone for 24h. RNA was extracted using TRIzol (Qiagen) as per standard protocol and total RNA was reverse transcribed using SuperScript II RT (Invitrogen). Expression levels of ACE-2 and TMPRSS2 were evaluated by SYBR real-time PCR using gene-specific primers as previously reported (Ma et al., Eye 2020;34:1212). GAPDH was used as an internal normalizer. The primers for GAPDH are as follows: 5'-GCCATCAATGACCCCTTCATT-3' (forward) and 5'-TTGACGGTGCCATGGAATTT-3' (reverse). Fold change in expression relative to that in untreated cells was determined using the standard ddCt method.

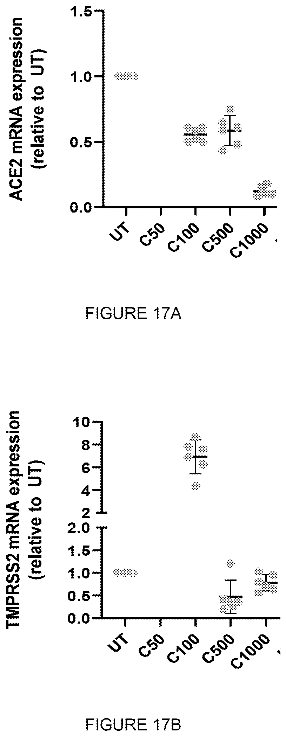

To assess the effect on ACE-1, ACE-2, TMPRSS2 and Furin expression levels, cells were pre-treated with L-carnitine concentrations ranging from 50 to 1,000 uM for 24 hr and ACE-1, ACE-2, TMPRSS2 and Furin mRNA levels were assessed. L-carnitine induced statistically significant downregulation of ACE-2 , mRNA as is shown in FIGURE 17A, while didn't not change the ACE-1 levels (not shown). The effects on TMPRSS2 are less conclusive. Based on the mRNA levels, TMPRSS2 seems to increase between 50-100uM (~1.5-4-fold) and decrease at 500uM by at least 75% as shown in FIGURE 17B. L-carnitine did not show any effect on the Furin expression levels (not shown).

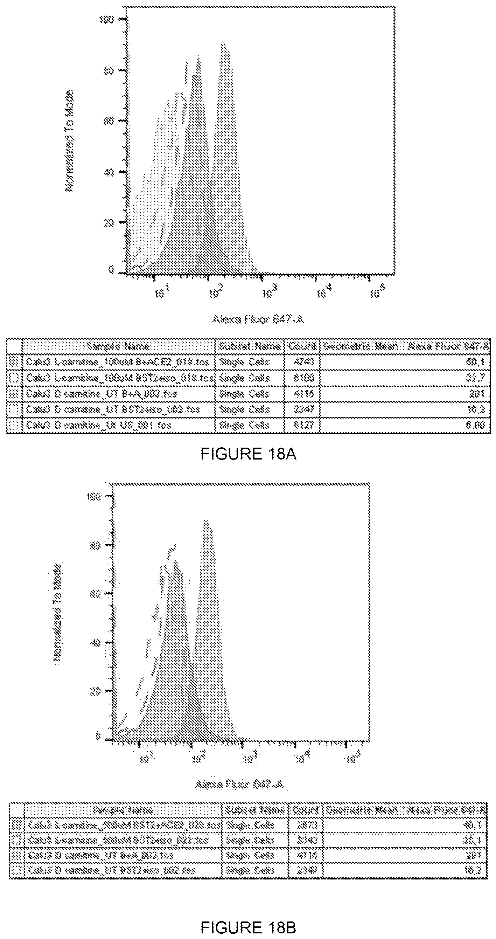

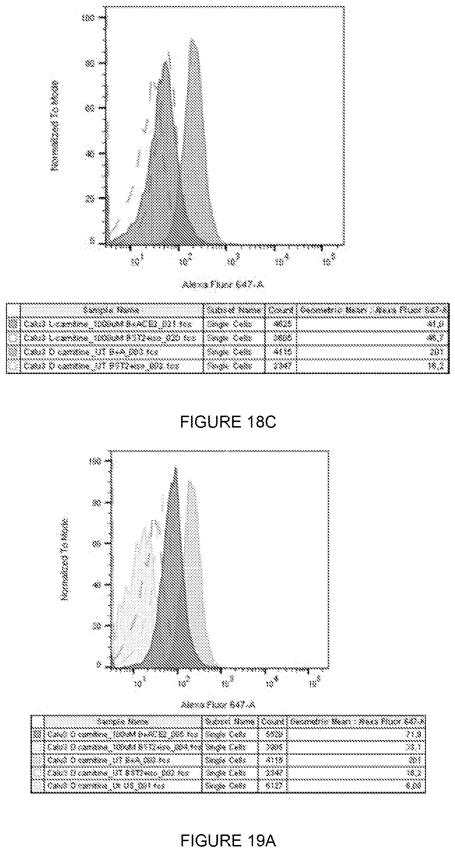

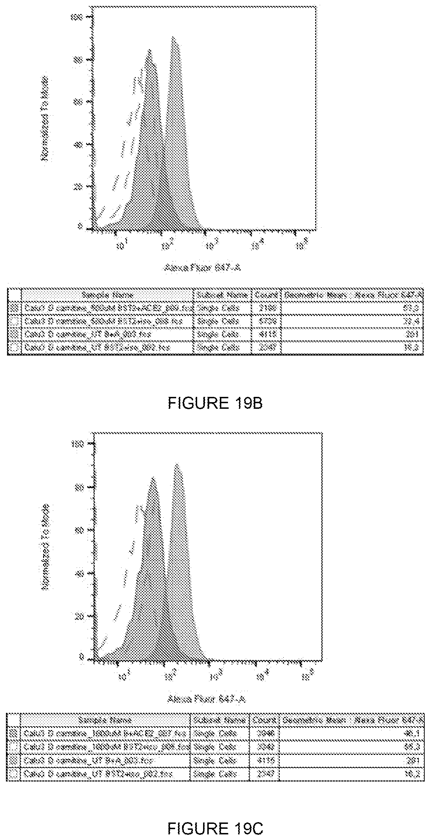

To assess the effects of L-carnitine on ACE-2 protein levels at the Calu-3 cell surface, and to have a better understanding of the mechanism of action, flow cytometry testing was conducted. Pre-treatment with 100, 500 and 1,000 uM of L-carnitine for 24-hr led to a significant decrease in the ACE-2 protein (about 75%) observed at 100 uM of L-carnitine treatment. The testing was repeated with D-Carnitine as well. Cells were either stained for ACE2 using either anti-ACE2 Ab, anti-BST2 Ab or the combination or both followed by secondary Ab isotype. UT-iso: untreated cells stained with anti-BST2 + the isotype control (orange dotted line), T-iso: treated cells stained with anti-BST2 + the isotype control (green dotted line), UT-αACE2: untreated cells stained with anti-ACE2 + anti-BST2 + isotype (orange area), T-αACE2: L-carnitine (Panel A) or D-carnitine-treated cells (Panel B) stained with anti-ACE2 + anti-BST2 + isotype (green area), and control cells Anti-BST2 + isotype (Grey area). The table represents the fluorescence counts. Interestingly, treatment with D-carnitine, the biologically inactive isomer of L-carnitine, also decreased significantly the ACE-2 protein levels but at a less extend than the L-carnitine. These data suggest that the effects of L-carnitine on downregulating ACE-2 are not dependent on its effects on fatty acid b-oxidation and energy generation. The results are shown in FIGURES 18A, 18B and 18C for L-carnitine and FIGURES 19A, 19B and 19C for D-carnitine.

### Infection of Calu-3 with SARS-CoV-2

SARS-CoV-2 which serves as the viral source was originally isolated from a COVID-19 patient in Quebec, Canada. The patient virus was amplified and tittered in Vero E6 using plaque assays (see below).

Calu-3, seeded in 12-well plate at 220,000 per well, were treated with 500uM and 1000uM Carnitine or with 1000 U/mL IFNα-2a as a positive control, shown previously to suppress SARS-CoV-2 infection. At 24h post stimulation, media was removed and cells were exposed to SARS-CoV-2 at MOI 0.01 and 0.1 in 350 µl in serum-free media. Cells plus media only were used as a control. After 3 h adsorption at 37°C, the viral inoculum was removed and cells were washed successively with 1XPBS and serum-free DMEM. New media (total 1mL) containing aforementioned concentrations of carnitine was then added to cells. Cell-free supernatant (250 µl) was removed at 12, 24 and 48h post infection. Carnitine was replenished for 1ml media at 24h post infection. Viral production in the supernatant was quantified using standard plaque assays.

### Plaque assays in Vero E6

Vero E6 cells (125,000 cells/well) were infected with up to six, serial ten-fold dilutions of SARS-CoV-2 for 1h at RT (200 µl infection volume). Cell monolayers were covered with overlaying media (final concentration: 0.6% agarose in DMEM with 5% FBS) and the plates were incubated undisturbed for 72 hr at 37°C. Subsequently, cell monolayers were fixed in 4% formaldehyde and stained with 0.25% crystal violet (prepared in 30% methanol). Plaques are counted and imaged using a DSLR camera, processed and. Virus titer is expressed as plaque-forming units(pfu)/µl and multiplicity of infection (MOI) expressed as: MOI = pfu of virus used for infection / number of cells.

### Dose-dependent inhibition of Calu-3 infection by L-carnitine

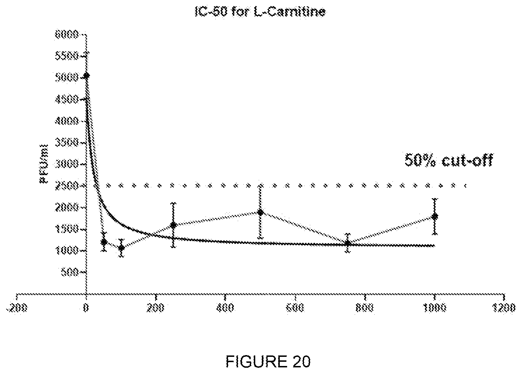

Cells were pre-treated with 50, 100, 250, 500, 750 and 1,000 uM L-carnitine for 24 hrs. The infection was performed at an MOI 0.01 as described above. Plaque assay was conducted in Vero cells after 24 and 48 hr and the plates were developed 65 hr post infection (A). IFNalpha2a I is used as a positive control. Experiments are the repeat of 9 biological and 12 technical replicates. Analysis was done using GraphPad Prism software (Version 8.4.3). Percentage of plaques in the presence of an antiviral was expressed relative to the virus alone group, which was set at 100%. Data were fit using a nonlinear regression model and the equation log inhibitor vs. variable slope (four parameters). The IC50 was calculated based on all experiments done at varying L-carnitine concentrations. Regression curve, performed by GraphPad Prism software (Version 8.4.3) indicates an IC50 of 138.8 uM (B) and is shown in FIGURE 20.

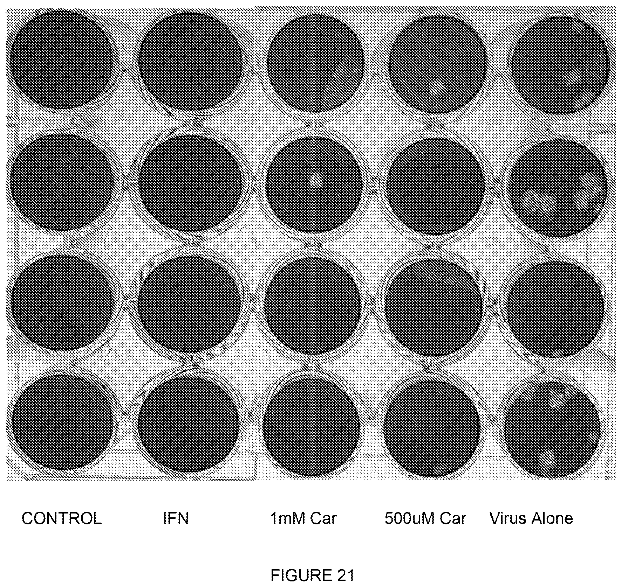

As is shown in FIGURE 21, which shows the 24 hour post infection at 10⁻² dilution. As can be seen, at both 1mM concentration of carnitine and 500 µM of carnitine was successful at inhibiting virus growth on the media.

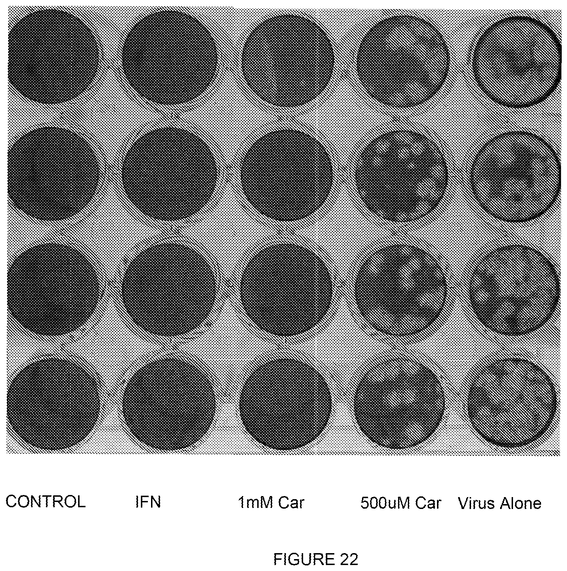

As is shown in FIGURE 22 which shows the 48 hour post infection at 10⁻² dilution. As can be seen, at both 1mM concentration of carnitine and 500 µM of carnitine was successful at inhibiting virus growth on the media versus the growth of the virus by itself. This data suggest that carnitine is effective in reducing the severity of the infection and can prevent growth of the virus at higher concentration.

### Discussion of Results

The forgoing Examples show the investigation of the impact of L-carnitine supplementation on preventing SARS-CoV-2 attachment, processing and subsequent infection once exposed to a mammal. It was discovered that ACE-2 as well as TMPRSS2 and Furin tissue and serum levels decreased significantly following an exercise-induced inflammation when the mammal diet was supplemented with L-Carnitine. It was further discovered that L-carnitine pre-treatment prevented Calu-3 infection by SARS-CoV-2, most likely by decreasing its attachment as demonstrated by a significant decrease in the ACE-2 surface cell availability. In addition, the decrease in TMPRSS2 and Furin protein levels in rodent and human tissues, suggests that L-carnitine supplementation can also impact the viral processing by inhibiting the cleavage of its spike protein. The latter was not reproduced in the Calu-3 cell culture for the Furin effect in particular. It is possible that L-carnitine may not impact this under these conditions and in the absence of a controlled inflammatory stimulus.

In addition, inflammatory disease states such as type II diabetes, hypertension, obesity and general aging and frailty are known to be major risk factors for SARS-CoV-2 induced mortality most likely though increased inflammation. The virus itself has been described to induce inflammation. ACE-2 levels in lungs and plasma are higher under inflammatory conditions. Plasma L-carnitine further is know to decrease with age and in inflammatory disease states.

As is shown in the Examples above, the exercise-induced inflammation also resulted in an increase in ACE-2, TMPRSS2, Furin and CRP serum levels, which was mitigated by L-carnitine supplementation. Both exhaustive aerobic and resistance exercise have been shown to increase short term (<96 hrs) oxidative stress and mechanical damage, resulting in increased inflammation. Exercise-induced inflammation can lead to elevations in ACE-2 which consequently reduces inflammation by generating the anti-inflammatory Angioten-(1-7). Indeed, increases in CRP concentration has been shown to be 168 % elevated above baseline after exercise and to peak at 2 days afterwards (253 % over baseline values). It has also reported that SARS-CoV-2 infection is associated with wide spread symptoms which include muscle weakness and pain. It was found that L-carnitine modulated ACE-1 and ACE-2 in the rodent lung and muscle tissues to keep the ACE-1/ACE-2 ratio in the normal range, suggesting that L-carnitine supplementation may help with the observed muscle weakness and in lung injuries seen in humans.

ACE-1 levels were unchanged in humans and decreased in rodents leading to a decreased ACE-1/ACE-2 ratio in rodents and no change in humans. These findings are important as an increase in the ACE-1/ACE-2 ratio is a marker of increased inflammation and ultimately the cytokine storm seen in advanced disease states.

Both our human and rodent data demonstrated a decrease in TMPRSS2 and Furin in lung, muscle, liver and plasma tissues, following exercise-induced inflammation. Activation of the spike protein by its cleavage by TMPRSS2 and Furin has been shown to be essential in viral cell entry.

The data in the Examples show an inhibition of the viral infection subsequent to a decrease in the ACE-2 cell surface levels in Calu-3 lung epithelial cells by L-carnitine. This was observed when the cells are pre-treated with L-carnitine for 24 hr. prior to infection. In addition, D-carnitine, the biologically inactive isomer of L-carnitine, also led to a decrease in the ACE-2 cell surface levels. In addition, the changes in the protein levels were mediated by decrease in the mRNA levels. This observation suggests that the effects of L-carnitine in decreasing ACE-2 are mediated through its gene regulation. The lack of effects on TMPRSS2 and Furin in the Calu-3 cells as compared to the rodent and human biomarkers, may be due to a lack of controlled inflammation in the cell culture. The testing in the Calu-3 cells was performed in the absence of an inflammatory stimulus.

A clinical study will is currently being commissioned to further demonstrate the benefits of carnitine supplementation of diet in preventing, treating, lessening of the severity of the infection in mammals caused by the SARS-CoV-2

These and other modifications and variations to the present invention may be practiced by those of ordinary skill in the art, without departing from the scope of appended claims. The foregoing description is by way of example only, and is not intended to limit the invention so further described in the appended claims.

## Claims

1. An angiotensin-converting enzyme 2 (ACE-2) reducing nutritional supplement comprising an ACE-2 reducing component for the use in the treatment, prevention, and/or delay of progression of infection in a mammal caused by an infectious agent which binds to the (ACE-2) in the mammal, wherein the infectious agent comprises a corona virus, wherein the ACE-2 reducing component is L-carnitine tartrate, and wherein said nutritional supplement comprises a) L-carnitine tartrate; and b) an auxiliary substance.

2. The nutritional supplement for use according to claim 1, wherein the ACE-2 reducing component is administered orally, nasally, intravenously, intramuscularly or transdermally to the mammal.

3. The nutritional supplement for use according to claim 1 or 2, further comprising administering the ACE-2 reducing component to the mammal, prior to the onset of an infection or during the infection from the infectious agent.

4. The nutritional supplement for use according to any one of claims 1-3, wherein the carnitine is administered to the mammal once or twice a day, and wherein the ACE-2 reducing component ACE-2 component is administered in an amount between about 5mg and 10,000 mg per day, typically between about 250 mg and 5000 mg per day, more typically between about 500 and 4000 mg per day.

5. The nutritional supplement for use according to any one of the preceding claims 1-4, wherein the corona virus is SARS-COV-2.

6. The nutritional supplement for use according to any one of claims 1-5, wherein the an auxiliary substance comprises at least one of excipients, binders, carriers, antiseptics, antioxidants, disintegrators, lubricants, flavoring agents, or a combination of two or more.

## Patentansprüche

1. Ein Angiotensin-konvertierendes Enzym 2 (ACE-2) reduzierendes Nahrungsergänzungsmittel, das eine ACE-2-reduzierende Komponente umfasst, zur Verwendung bei der Behandlung, Vorbeugung und/oder Verzögerung des Fortschreitens einer Infektion bei einem Säugetier, die durch einen an das (ACE-2) des Säugetiers bindenden Infektionserreger verursacht wird, wobei der Infektionserreger ein Coronavirus umfasst, wobei die ACE-2-reduzierende Komponente L-Carnitintartrat ist, und wobei das Nahrungsergänzungsmittel a) L-Carnitintartrat und b) eine Hilfssubstanz umfasst.

2. Das Nahrungsergänzungsmittel zur Verwendung nach Anspruch 1, wobei die ACE-2-reduzierende Komponente dem Säugetier oral, nasal, intravenös, intramuskulär oder transdermal verabreicht wird.

3. Das Nahrungsergänzungsmittel zur Verwendung nach Anspruch 1 oder 2, weiter umfassend die Verabreichung der ACE-2-reduzierenden Komponente an das Säugetier vor dem Ausbruch einer Infektion oder während der Infektion durch den Infektionserreger.

4. Das Nahrungsergänzungsmittel zur Verwendung nach einem der Ansprüche 1-3, wobei das Carnitin dem Säugetier einmal oder zweimal täglich verabreicht wird und wobei die ACE-2-reduzierende Komponente in einer Menge zwischen etwa 5 mg und 10.000 mg pro Tag, typischerweise zwischen etwa 250 mg und 5.000 mg pro Tag, ganz typisch zwischen etwa 500 mg und 4.000 mg pro Tag verabreicht wird.

5. Das Nahrungsergänzungsmittel zur Verwendung nach einem der vorstehenden Ansprüche 1-4, wobei es sich bei dem Coronavirus um SARS-CoV-2 handelt.

6. Das Nahrungsergänzungsmittel zur Verwendung nach einem der Ansprüche 1-5, wobei die Hilfssubstanz mindestens eines von Hilfsstoffen, Bindemitteln, Trägerstoffen, Antiseptika, Antioxidantien, Sprengmitteln, Gleitmitteln, Aromastoffen oder eine Kombination aus zwei oder mehr umfasst.

## Revendications

1. Complément alimentaire réduisant l'enzyme de conversion de l'angiotensine 2 (ACE-2), comprenant un composant réduisant l'ACE-2, destiné à traiter, prévenir et/ou retarder la progression d'une infection chez un mammifère causée par un agent infectieux qui se lie à l'ACE-2 chez le mammifère, dans lequel l'agent infectieux comprend un coronavirus, dans lequel le composant réduisant de l'ACE-2 est le tartrate de L-carnitine, et dans lequel ledit complément alimentaire comprend a) du tartrate de L-carnitine ; et b) une substance auxiliaire.

2. Complément alimentaire à utiliser selon la revendication 1, dans lequel le composant réduisant l'ACE-2 est administré par voie orale, nasale, intraveineuse, intramusculaire ou transdermique au mammifère.

3. Complément alimentaire à utiliser selon la revendication 1 ou 2, comprenant en outre l'administration du composant réduisant l'ACE-2 au mammifère, avant le début d'une infection ou pendant l'infection par l'agent infectieux.

4. Complément alimentaire à utiliser selon l'une quelconque des revendications 1-3, dans lequel la carnitine est administrée au mammifère une ou deux fois par jour, et dans lequel le composant réduisant d'ACE-2 est administré en une quantité comprise entre environ 5 mg et 10 000 mg par jour, généralement entre environ 250 mg et 5 000 mg par jour, plus généralement entre environ 500 mg et 4 000 mg pajour.

5. Complément alimentaire destiné à être utilisé selon n'importe laquelle des revendications précédentes 1-4, dans lequel le coronavirus est le SARS-CoV-2.

6. Complément alimentaire à utiliser selon l'une quelconque des revendications 1-5, dans lequel une substance auxiliaire comprend au moins un élément parmi des excipients, des liants, des supports, des antiseptiques, des antioxydants, des agents désintégrants, des lubrifiants, des agents aromatisants ou une combinaison de deux ou plus.
